Europäisches Patentamt

European Patent Office

Office européen des brevets

(19)

(11) Publication number: **0 022 342**
B1

(12) **EUROPEAN PATENT SPECIFICATION**

(45) Date of publication of patent specification: **15.06.83**

(51) Int. Cl.³: **A 61 K 9/08, A 61 K 31/63**

(21) Application number: **80302189.8**

(22) Date of filing: **30.06.80**

(54) Sulfonamide solutions.

(30) Priority: **05.07.79 US 54546**

(43) Date of publication of application:
**14.01.81 Bulletin 81/2**

(45) Publication of the grant of the patent:
**15.06.83 Bulletin 83/24**

(84) Designated Contracting States:
**AT BE CH DE FR GB IT LI LU NL SE**

(56) References cited:
**DE - A - 2 627 706**
**DE - A - 2 631 779**
**FR - A - 2 340 100**
**GB - A - 1 538 903**
**US - A - 3 985 876**
**US - A - 4 018 889**

**The file contains technical information
submitted after the application was filed and not
included in this specification**

(73) Proprietor: **PFIZER INC.**
**235 East 42nd Street**
**New York, N.Y. 10017 (US)**

(72) Inventor: **Armstrong, William Wellesley**
**Horseshoe Road Mill Neck**
**Nassau, New York (US)**

(74) Representative: **Wood, David John et al,**
**Pfizer Limited Ramsgate Road**
**Sandwich Kent CT13 9NJ (GB)**

## Sulfonamide solutions

This invention relates to sulfonamide solutions suitable for pharmaceutical use. More particularly, it relates to aqueous parenteral solutions of sulfamethoxazole or sulfadoxine with trimethoprim in 2-pyrrolidone.

Antibacterial compositions containing sulfamethoxazole and trimethoprim are disclosed in U.S. Patent No. 3,515,783.

Parenteral solutions of sulfamethoxazole and trimethoprim containing the following water-miscible solvents are disclosed in U.S. Patent No. 3,551,564: polyethyleneglycol ethers of tetrahydrofurfuryl alcohol, dimethylformamide, dimethylsulfoxide, dimethylacetamide, diethylacetamide, 1,2-propyleneglycol, di-(1,2-propyleneglycol), 1,3-butylene glycol, glycerol formal, diethylene glycol and polyethylene glycol containing 2—15 ethylene oxide groups.

U.S. Patent No. 3,985,876 discloses solutions of trimethoprim with various sulfonamides, including sulfamethoxazole and sulfadoxine in a mixture of 10—60% water and 30—90% organic solvents such as dimethyl and diethylacetamide, dimethyl formamide, dimethylsulfoxide, glycerol formal and various glycols.

U.S. Patent No. 4,018,889 discloses oxytetracycline solutions containing from about 1 to 40% oxytetracycline in an aqueous vehicle containing from about 10 to 50% by weight of 2-pyrrolidone, about 0.8 to 1.3 molar proportions of a pharmaceutically acceptable magnesium compound soluble in the said solution, said solution having a pH value in the range of from about 7.5 to 9.5.

French patent publication No. 2340100 discloses stable injectable sulphonamide preparations at pH 5.5—7.5 comprising a solution of the diethanolamine salt of a sulphonamide (e.g. sulfafurzole) and a salt of a sulphonemide potentiator (e.g. diaveridine or trimethoprim lactate) in a mixture of water, propylene glycol and N-methylpyrrolidone in stated proportions.

German Offenlegungsschrift No. 2631779 discloses clear aqueous sulfonamide — trimethoprim solutions, characterised by containing a water-soluble sulphonamide salt, trimethoprim and polyvinylpyrrolidone with a K-value of 10—18 in water.

British Patent No. 1,538,903 discloses a composition comprising a physiologically active agent (e.g. a sulphonamide) or cosmetic agent and a topical pharmaceutical carrier composition containing at least 10% by weight of the carrier composition of a vehicle system comprising a mixture of 2-pyrrolidone and N-methyl-2-pyrrolidone in a weight ratio of between 1:4 and 4:1.

This invention provides a sulfonamide solution comprising (a) water (b) from 10 to 20% w/v based on the total volume of solution of sulfamethoxazole or sulfadoxine, (c) from 3 to 5% w/v based on the total volume of solution of trimethoprim and (d) from 60 to 80% w/v based on the total volume of solution of 2-pyrrolidone, the solution having a pH of from 5 to 9.5.

The sulfonamides utilized in the compositions of this invention are sulfamethoxazole and sulfadoxine. They are present in concentrations of from 10 to 20% w/v. The preferred concentration is 20% w/v.

Sulfamethoxazole [N$^1$-(5-methyl-3-isoxazolyl)sulfonilamide] is particularly described in U.S. Patent No. 2,888,455.

Sulfadoxine [N'-(5,6-dimethoxy-4-pyrimidyl)sulfonilamide] is described in U.S. Patent No. 3,132,139.

Trimethoprim is also utilized in the compositions of this invention as a potentiator in concentrations of from 3 to 5% w/v. The preferred concentration is 4% w/v.

Trimethoprim [2,4-diamino-5-(3,4,5-trimethoxybenzyl)pyrimidine] is described in U.S. Patent No. 2,909,522.

2-Pyrrolidone is present as a co-solvent for the sulfonamide and trimethoprim in a concentration of from 60 to 80% w/v.

2-Pyrrolidone is also known as 2-pyrrolidinone, 2-oxopyrrolidine, alpha-pyrrolidone and 2-keto-pyrrolidine. It has an oral LD$_{50}$ of 8 g/kg in rats and 3.8 g/kg by intraperitoneal injection in mice.

The pH value of these compositions is adjusted if necessary to pH 5 to 9.5. The pH can be adjusted by means of a base that is pharmaceutically acceptable, such as monoethanolamine.

As an optional ingredient polyvinylpyrrolidone, having a molecular weight of between 5,000 and 100,000 (K-12 to 30), may also be present in these compositions in a concentration of from 1 to 5% w/v. The polyvinylpyrrolidone most preferred for this invention is one having an average molecular weight of 10,000—17,000 (where K-value = 17). It is present in part as a cosolubilizer and may improve tissue toleration.

As optional cosolvents ingredients such as propylene glycol, polyethylene glycols, benzyl alcohol, dimethylacetamide, ethyl lactate and glycerol formal may be present at concentrations of from 0.5 to 30% w/v.

The stability of these solutions for therapeutic administration is still further enhanced by use of an antioxidant such as monothioglycerol at levels of from 0.1 to 1% w/v.

The primary application of these sulfonamide compositions is as a parenteral composition but the new compositions can also be used for topical application.

These solutions produce efficacious blood levels and are well tolerated in contrast to the short term blood levels and tissue damage routinely experienced with presently available highly alkaline aqueous sulfonamide solutions.

The sulfonamide antibiotic compositions of this invention are also easy to syringe over a wide temperature range and are satisfactory from a physical and chemical stability standpoint. The compositions of this invention are preferably prepared by mixing the 2-pyrrolidone with water and other cosolvents, if present. The sulfonamide and trimethoprim are then added and stirred until a clear solution results. The pH is then adjusted to the desired range. If polyvinylpyrrolidone is to be included, it is added to the water at the time of mixing the 2-pyrrolidone.

The present invention is illustrated by the following Examples:

### Example 1—3

| | g/100 ml | | |
| --- | --- | --- | --- |
| | Example 1 | Example 2 | Example 3 |
| Sulfamethoxazole | 20.00 | 20.00 | 20.00 |
| Trimethoprim | 4.00 | 4.00 | 4.00 |
| 2-Pyrrolidone | 60.00 | 60.00 | 60.00 |
| Dimethylacetamide | — | — | 10.00 |
| Polyvinylpyrrolidone (K = 17) | 5.00 | 5.00 | — |
| Glycerol formal | — | 20.00 | — |
| Benzyl alcohol | 0.90 | 0.90 | 0.90 |
| Monothioglycerol | 0.20 | 0.20 | 0.20 |
| Monoethanolamine | 4.50 | 4.50 | — |
| Water q.s. to | 100.00 | 100.00 | 100.00 |
| pH | 8.8 | 8.8 | 7.00 |
| Viscosity cts at 25°C | 35 | 31 | 28 |

The above solutions were prepared by dissolving all the ingredients in water, with the sulfamethoxazole and trimethoprim being added last.

Raising the pH of Examples 1 and 2 with monoethanolamine to pH 9.5 and 8.8 respectively produced similar solutions with no significant viscosity changes.

Examples 4—5

| | g/100 ml | |
|---|---|---|
| | Example 4 | Example 5 |
| Sulfadoxine | 20.00 | 20.00 |
| Trimethoprim | 4.00 | 4.00 |
| 2-Pyrrolidone | 60.00 | 70.00 |
| Polyvinylpyrrolidone (K = 17) | 5.00 | 5.00 |
| Dimethylacetamide | 10.00 | 10.00 |
| Glycerol formal | 7.50 | 7.50 |
| Benzyl alcohol | 0.90 | 0.90 |
| Monothioglycerol | 0.20 | 0.20 |
| Monoethanolamine | 2.00 | — |
| Water q.s. to | 100.00 | 100.00 |
| pH | 8.7 | 7.7 |
| Viscosity cts at 25°C | 49 | 53 |

The above solutions were prepared by dissolving all the ingredients in water, with the sulfadoxine and trimethoprim being added last.

Examples 6—8

| | g/100 ml | | |
|---|---|---|---|
| | Example 6 | Example 7 | Example 8 |
| Sulfadoxine | 20.00 | 20.00 | 20.00 |
| Trimethoprim | 4.00 | 4.00 | 4.00 |
| 2-Pyrrolidone | 80.00 | 80.00 | 70.00 |
| Polyvinylpyrrolidone (K = 17) | 5.00 | — | — |
| Dimethylacetamide | 10.00 | 10.00 | 10.00 |
| Benzyl alcohol | 0.90 | 0.90 | 0.90 |
| Monothioglycerol | 0.20 | 0.20 | 0.20 |
| Monoethanolamine | — | — | 2.00 |
| Water q.s. to | 100.00 | 100.00 | 100.00 |
| pH | 7.9 | 8.0 | 8.4 |
| Viscosity cts at 25°C | 63 | 28 | 26 |

The above solutions were prepared by dissolving all the ingredients in water, with the sulfadoxine and trimethoprim being added last.

**0 022 342**

**Claims for the contracting states: BE, CH, DE, FR, GB, iT, Li, Lu, NL, SE**

1. A sulfonamide solution comprising (a) water (b) from 10 to 20% w/v based on the total volume of solution of sulfamethoxazole or sulfadoxine (c) from 3 to 5% w/v based on the total volume of solution of trimethoprim and (d) from 60 to 80% w/v based on the total volume of solution of 2-pyrrolidone, the solution having a pH of from 5 to 9.5.

2. A solution as claimed in claim 1 wherein the solfonamide is sulfamethoxazole.

3. A solution as claimed in claim 1 wherein the sulfonamide is sulfadoxine.

4. A solution as claimed in any one of the preceding claims comprising polyvinylpyrrolidone having a m.w. of from 5000—100,000 in a concentration of from 1 to 5% w/v based on the total volume of solution.

5. A sulfonamide solution comprising (a) water (b) 20% w/v based on the total volume of solution of sulfamethoxazole, (c) 4% w/v based on the total volume of solution of trimethoprim, (d) from 60 to 80% w/v based on the total volume of solution of 2-pyrrolidone, and (e) from 1 to 5% w/v based on the total volume of solution of polyvinylpyrrolidone having a m.w. of from 5000—100,000, the solution having a pH of from 7 to 9.5.

6. A sulfonamide solution comprising (a) water (b) 20% w/v based on the total volume of solution of sulfadoxine (c) 4% w/v based on the total volume of solution of trimethoprim, (d) from 60% to 80% w/v based on the total volume of solution of 2-pyrrolidone, and (e) from 1 to 5% w/v based on the total volume of solution of polyvinylpyrrolidone having a m.w. of from 5000—100,000, the solution having a pH of from 7 to 9.5.

7. A solution as claimed in any one of the preceding claims which contains propylene glycol, a polyethylene glycol, benzyl alcohol, dimethylacetamide, ethyl lactate or glycerol formal in an amount of from 0.5 to 30% w/v based on the total volume of solution.

**Claims for the contracting state: AT**

1. A process for preparing a sulfonamide solution characterised by forming a solution comprising (a) water (b) from 10 to 20% w/v based on the total volume of solution of sulfamethoxazole or sulfadoxine (c) from 3 to 5% w/v based on the total volume of solution of trimethoprim and (d) from 60 to 80% w/v based on the total volume of solution of 2-pyrrolidone, the solution having a pH of from 5 to 9.5.

2. A process as claimed in claim 1 wherein the sulfonamide is sulfamethoxazole.

3. A process as claimed in claim 1 wherein the sulfonamide is sulfadoxine.

4. A process as claimed in any one of the preceding claims wherein polyvinylpyrrolidone having a m.w. of from 5000—100,000 in a concentration of from 1 to 5% w/v based on the total volume of solution is incorporated in the solution.

5. A process for preparing a solfonamide solution characterised by forming a solution comprising (a) water (b) 20% w/v based on the total volume of solution of sulfadoxine (c) 4% w/v based on the total the total volume of solution of trimethoprim, (d) from 60 to 80% w/v based on the total volume of solution of 2-pyrrolidone, and (e) from 1 to 5% w/v based on the total volume of solution of polyvinylpyrrolidone having a m.w. of from 5000—100,000, the solution having a pH of from 7 to 9.5.

6. A process for preparing a sulfonamide solution characterised by forming a solution comprising (a) water (b) 20% w/v based on the total volume of solution of sulfadoxine (c) 4% w/v based on the total volume of solution of trimethoprim, (d) from 60% to 80% 2/v based on the total volume of solution of 2-pyrrolidone, and (e) from 1 to 5% 2/v based on the total volume of solution of polyvinylpyrrolidone having a m.w. of from 5000—100,000, the solution having a pH of from 7 to 9.5.

7. A process as claimed in any one of the preceding claims wherein an additional co-solvent selected from propylene glycol, a polyethylene glycol, benzyl alcohol, dimethylacetamide, ethyl lactate and glycerol formal is incorporated in the solution in an amount of from 0.5 to 30% w/v based on the total volume of solution.

8. A process as claimed in any one of the preceding claims wherein the sulphamethoxazole or sulfadoxine and trimethoprim are dissolved in the aqueous 2-pyrrolidone, said 2-pyrrolidone containing the polyvinylpyrrolidone or additional co-solvent, if present, and if necessary adjusting the pH to from 5 to 9.5.

**Revendications pour les Etats contractants: BE, CH, DE, FR, GB, IT, LI, LU, NL**

1. Solution de sulfamide, comprenant (a) de l'eau, (b) 10 à 20% en poids/volume, sur la base du volume total de solution, de sulfaméthoxazole ou de sulfadoxine, (c) 3 à 5% en poids/volume, sur la base du volume total de solution, de triméthoprim et (d) 60 à 80% en poids/volume, sur la base du volume total de solution, de 2-pyrrolidone, la solution ayant un pH de 5 à 9,5.

2. Solution suivant la revendication 1, dans laquelle le sulfamide est le sulfaméthoxazole.

3. Solution suivant la revendication 1, dans laquelle le sulfamide est la sulfadoxine.

4. Solution suivant l'une quelconque des revendications précédentes, comprenant de la poly-

5

vinylpyrrolidone de poids moléculaire compris entre 5000 et 100 000 à une concentration de 1 à 5% en poids/volume sur la base du volume total de la solution.

5. Solution de sulfamide, comprenant (a) de l'eau, (b) 20% en poids/volume, sur la base du volume total de solution, de sulfaméthoxazole, (c) 4% en poids/volume, sur la base du volume total de solution, de triméthoprim, (d) 60 à 80% en poids/volume, sur la base du volume total de solution, de 2-pyrrolidone et (e) 1 à 5% en poids/volume, sur la base du volume total de solution, de polyvinylpyrrolidone ayant un poids moléculaire de 5000 à 100 000, la solution ayant un pH de 7 à 9,5.

6. Solution de sulfamide, comprenant (a) de l'eau, (b) 20% en poids/volume, sur la base du volume total de solution, de sulfadoxine, (c) 4% en poids/volume, sur la base du volume total de solution, de triméthoprim, (d) 60 à 80% en poids/volume, sur la base du volume total de solution, de 2-pyrrolidone et (e) 1 à 5% en poids/volume, sur la base du volume total de solution, de polyvinylpyrrolidone ayant un poids moléculaire de 5000 à 100 000, la solution ayant un pH de 7 à 9,5.

7. Solution suivant l'une quelconque des revendications précédentes, qui contient du propylène-glycol, un polyéthylène-glycol, de l'alcool benzylique, du diméthylacétamide, du lactate d'éthyle ou du formal du glycérol en une quantité de 0,5 à 30% en poids/volume sur la base du volume total de solution.

**Revendications pour l'Etat contractant: AT**

1. Procédé de préparation d'une solution de sulfamide, caractérisé par la formation d'une solution comprenant (a) de l'eau, (b) 10 à 20% en poids/volume, sur la base du volume total de solution, de sulfaméthoxazole ou de sulfadoxine, (c) 3 à 5% en poids/volume, sur la base du volume total de solution, de triméthoprim et (d) 60 à 80% en poids/volume, sur la base du volume total de solution, de 2-pyrrolidone, la solution ayant un pH de 5 à 9,5.

2. Procédé suivant la revendication 1, dans lequel le sulfamide est le sulfaméthoxazole.

3. Procédé suivant la revendication 1, dans lequel le sulfamide est la sulfadoxine.

4. Procédé suivant l'une quelconque des revendications précédentes, dans lequel une polyvinylpyrrolidone ayant un poids moléculaire de 5000 à 100 000, à une concentration de 1 à 5% en poids/volume, sur la base du volume total de solution, est incorporée à la solution.

5. Procédé de préparation d'une solution de sulfamide, caractérisé par la formation d'une solution comprenant (a) de l'eau, (b) 20% en poids/volume, sur la base du volume total de solution, de sulfaméthoxazole, (c) 4% en poids/volume, sur la base du volume total de solution, de triméthoprim, (d) 60 à 80% en poids/volume, sur la base du volume total de solution, de 2-pyrrolidone et (e) 1 à 5% en poids/volume, sur la base du volume total de solution, de polyvinylpyrrolidone ayant un poids moléculaire de 5000 à 100 000, la solution ayant un pH de 7 à 9,5.

6. Procédé de préparation d'une solution de sulfamide, caractérisé par la formation d'une solution comprenant (a) de l'eau, (b) 20% en poids/volume, sur la base du volume total de solution, de sulfadoxine, (c) 4% en poids/volume, sur la base du volume total de solution, de triméthoprim, (d) 60 à 80% en poids/volume, sur la base du volume total de solution, de 2-pyrrolidone et (e) 1 à 5% en poids/volume, sur la base du volume total de solution, de polyvinylpyrrolidone ayant un poids moléculaire de 5000—100 000, la solution ayant un pH de 7 à 9,5.

7. Procédé suivant l'une quelconque des revendications précédentes, dans lequel un cosolvant additionnel choisi entre le propylène-glycol, un polyéthylène-glycol, l'alcool benzylique, le diméthylacétamide, le lactate d'éthyle et le formal du glycérol est incorporé à la solution en une quantité de 0.5 à 30% en poids/volume, sur la base du volume total de solution.

8. Procédé suivant l'une quelconque des revendications précédentes, dans lequel le sulfaméthoxazole ou la sulfadoxine et le triméthoprim sont dissous dans la 2-pyrrolidone aqueuse, ladite 2-pyrrolidone contenant la polyvinylpyrrolidone ou un cosolvant additionnel éventuellement présent, et, le cas échéant, le pH est ajusté à une valeur de 5 à 9,5.

**Patentansprüche fur die Vertragsstaaten: BE, CH, DE, FR, GB, IT, LI, LU, NL**

1. Eine Sulfonamidlösung enthaltend (a) Wasser (b) 10 bis 20% Gewicht/Volumen bezogen auf das Gesamtvolumen der Lösung an Sulfamethoxazol oder Sulfadoxin, (c) 3 bis 5% Gewicht/Volumen bezogen auf das gesamte Volumen der Lösung an Trimethoprim und (d) 60 bis 80% Gewicht/Volumen bezogen auf das Gesamtvolumen der Lösung an 2-Pyrrolidon, wobei die Lösung einen pH-Wert von 5 bis 9,5 aufweist.

2. Lösung gemäß Anspruch 1 in welcher das Sulfonamid Sulfamethoxazol ist.

3. Lösung gemäß Anspruch 1 in welcher das Sulfonamid Sulfadoxin ist.

4. Lösung gemäß einem der vorgehenden Ansprüche enthaltend Polyvinylpyrrolidon mit einem Molekulargewicht von 5000 bis 100 000 in einer Konzentration von 1 bis 5% Gewicht/Volumen bezogen auf das Gesamtvolumen der Lösung.

5. Eine Sulfonamidlösung enthaltend (a) Wasser (b) 20% Gewicht/Volumen bezogen auf das Gesamtvolumen der Lösung an Sulfamethoxazol, (c) 4% Gewicht/Volumen bezogen auf das Gesamt-volumen der Lösung an Trimethoprim, (d) 60 bis 80% Gewicht/Volumen bezogen auf das Gesamtvolumen der Lösung an 2-Pyrrolidon und (e) 1 bis 5% Gewicht/Volumen bezogen auf das Gesamtvolumen der Lösung an Polyvinylpyrrolidon mit einem Molekulargewicht von 5000 bis 100 000, wobei die Lösung einen pH-Wert von 7 bis 9,5 aufweist.

6. Eine Sulfonamidlösung enthaltend (a) Wasser (b) 20% Gewicht/Volumen bezogen auf das Gesamtvolumen der Lösung an Sulfadoxin, (c) 4% Gewicht/Volumen bezogen auf des Gesamtvolumen der Lösung an Trimethoprim, (d) 60 bis 80% Gewicht/Volumen bezogen auf das Gesamtvolumen der Lösung an 2-Pyrrolidon und (e) 1 bis 5% Gewicht/Volumen bezogen auf das Gesamtvolumen der Lösung an Polyvinylpyrrolidon mit einem Molekulargewicht von 5000 bis 100 000, wobei die Lösung einen pH-Wert von 7 bis 9,5 aufweist.

7. Lösung gemäß einem der vorgehenden Ansprüche enthaltend Propylenglycol, ein Polyäthylenglycol, Benzylalkohol, Dimethylacetamid, Äthyllactat oder Glycerinformal in einer Menge von 0,5 bis 30% Gewicht/Volumen bezogen auf das Gesamtvolumen der Lösung.

**Patentansprüche für den Vertragsstaat: AT**

1. Verfahren zur Herstellung einer Sulfonamidlösung, dadurch gekennzeichnet, daß man eine Lösung bildet, die (a) Wasser, (b) 10 bis 20% Masse/Vol., bezogen auf das Gesamtvolumen der Lösung, Sulfamethoxazol oder Sulfadoxin, (c) 3 bis 5% Masse/Vol., bezogen auf das Gesamtvolumen der Lösung, Trimethoprim und (d) 60 bis 80% Masse/Vol., bezogen auf das Gesamtvolumen der Lösung, 2-Pyrrolidon umfaßt, wobei die Lösung einen pH von 5 bis 9,5 aufweist.

2. Verfahren, wie in Anspruch 1 beansprucht, wobei das Sulfonamid Sulfamethoxazol ist.

3. Verfahren, wie in Anspruch 1 beansprucht, wobei das Sulfonamid Sulfadoxin ist.

4. Verfahren, wie in einem der vorhergehenden Ansprüche beansprucht, wobei Polyvinylpyrrolidon mit einer Molmasse von 5000 bis 100 000 in einer Konzentration von 1 bis 5% Masse/Vol., bezogen auf das Gesamtvolumen der Lösung, der Lösung einverleibt wird.

5. Verfahren zur Herstellung einer Sulfonamidlösung, dadurch gekennzeichnet, daß man eine Lösung bildet, die (a) Wasser, (b) 20% Masse/Vol., bezogen auf das Gesamtvolumen der Lösung, Sulfamethoxazol, (c) 4% Masse/Vol., bezogen auf das Gesamtvolumen der Lösung, Trimethoprim, (d) 60 bis 80% Masse/Vol, bezogen auf das Gesamtvolumen der Lösung, 2-Pyrrolidon und (e) 1 bis 5% Masse/Vol., bezogen auf das Gesamtvolumen der Lösung, Polyvinylpyrrolidon mit einer Molmasse von 5000 bis 100 000 umfaßt, wobei die Lösung einen pH von 7 bis 9,5 aufweist.

6. Verfahren zur Herstellung einer Sulfonamidlösung, dadurch gekennzeichnet, daß man eine Lösung bildet, die (a) Wasser, (b) 20% Masse/ Vol., bezogen auf das Gesamtvolumen der Lösung, Sulfadoxin, (c) 4% Masse/Vol., bezogen auf das Gesamtvolumen der Lösung, Trimethoprim, (d) 60 bis 80% Masse/Vol., bezogen auf das Gesamtvolumen der Lösung, 2-Pyrrolidon und (e) 1 bis 5% Masse/Vol., bezogen auf das Gesamtvolumen der Lösung, Polyvinylpyrrolidon mit einer Molmasse von 5000 bis 100 000 umfaßt, wobei die Lösung einen pH von 7 bis 9,5 aufweist.

7. Verfahren, wie in einem der vorhergehenden Ansprüche beansprucht, wobei ein zusätzliches Colösungsmittel ausgewählt unter Propylenglykol, einem Polyäthylenglykol, Benzylalkohol, Dimethylacetamid, Äthyllactat und Glycerinformal der Lösung in einem Anteil von 0,5 bis 30% Masse/Vol., bezogen auf das Gesamtvolumen der Lösung, einverleibt wird.

8. Verfahren, wie in einem der vorhergehenden Ansprüche beansprucht, wobei das Sulfamethoxazol oder Sulfadoxin und Trimethoprim im wässerigen 2-Pyrrolidon gelöst werden, wobei das 2-Pyrrolidon das Polyvinylpyrrolidon oder zusätzliches Colösungsmittel, wenn vorhanden, enthält und, wenn notwendig, der pH auf 5 bis 9,5 eingestellt wird.